**Europäisches Patentamt**

**European Patent Office**

**Office européen des brevets**

(19)

(11) Veröffentlichungsnummer: **0 082 277**
**A1**

(12) # EUROPÄISCHE PATENTANMELDUNG

(21) Anmeldenummer: **82110061.7**

(22) Anmeldetag: **30.10.82**

(51) Int. Cl.³: **C 07 C 31/18**
C 07 C 29/136, C 07 C 29/14

(30) Priorität: **07.11.81 DE 3144320**

(43) Veröffentlichungstag der Anmeldung:
**29.06.83 Patentblatt 83/26**

(84) Benannte Vertragsstaaten:
**AT BE CH DE FR GB IT LI LU NL SE**

(71) Anmelder: **Henkel Kommanditgesellschaft auf Aktien**
**Postfach 1100 Henkelstrasse 67**
**D-4000 Düsseldorf 1(DE)**

(72) Erfinder: **Lepper, Herbert, Dr.**
**Gauweg 10**
**D-5000 Köln-Mühlheim(DE)**

(72) Erfinder: **Schütt, Hartwig, Dr.**
**Haydnstrasse 50**
**D-4000 Düsseldorf-Benrath(DE)**

(54) **Verfahren zur kontinuierlichen Herstellung von mehrwertigen Alkoholen.**

(57) Bei der kontinuierlichen Herstellung von mehrwertigen Alkoholen (Zuckeralkoholen) durch Hydrierung von Kohlehydraten in Gegenwart von Ruthenium enthaltenden Katalysatoren bei erhöhter Temperatur und erhöhtem Druck wird mit Vorteil ein Katalysatorfestbett aus stückigen Ruthenium-Trägerkatalysatoren eingesetzt.

EP 0 082 277 A1

Henkelstrasse 67          **Henkel KGaA**
4000 Düsseldorf         ZR-FE/Patente

Düsseldorf, den 5.11.1981        Dr. Gla/Br

P a t e n t a n m e l d u n g

D 6436    EP

"Verfahren zur kontinuierlichen Herstellung von mehrwertigen Alkoholen"

Die Erfindung betrifft ein verbessertes Verfahren zur
Herstellung von mehrwertigen Alkoholen durch katalytische Hydrierung von Kohlehydraten in Gegenwart von
Ruthenium-Trägerkatalysatoren.

Im Zusammenhang mit dieser Erfindung umfaßt der Begriff
"Kohlehydrat" Monosaccharide und Polysaccharide, wobei
es sich sowohl um reine Verbindungen wie Glucose, Fructose und Saccharose als auch um Saccharidgemische, wie
zum Beispiel Stärkehydrolysate handeln kann. Der Begriff
"Polysaccharid" bezeichnet Saccharide mit mehr als einer
Monosaccharideinheit, also auch Di-, Tri- und Oligosaccharide.

Die Überführung von Kohlehydraten in mehrwertige Alkohole (Zuckeralkohole) unter Hydrierbedingungen und in
Gegenwart von Ruthenium auf festen Trägermaterialien ist
bekannt. So wurde in der US-PS 2 868 897 ein Verfahren
zur katalytischen Hydrierung von Kohlehydraten vorgeschlagen, bei dem man Ruthenium auf inerten Trägermaterialien wie Kohlenstoff, Aluminiumoxid, Siliciumdioxid, Kieselgur, Kieselgel oder Diatomeenerde als
Katalysator benutzt. Als Kohlehydrate wurden hier
Monosaccharide wie Glucose und Fructose und Disaccharide

wie Saccharose und Lactose eingesetzt. So wurden aus Glucose Sorbit und aus Saccharose und Lactose unter gleichzeitiger Hydrolyse die entsprechenden Hexite erhalten.

Die Verwendung von Ruthenium auf Tierkohle als Katalysator bei der Hydrierung von Monosacchariden wird auch von N.A.. Vasyunina et al in Izv. Akad. Nauk. SSR Khimicheskaya 4 (1969) S. 848-854 beschrieben. Verfahren zur katalytischen Hydrierung von Kohlehydraten in Gegenwart von Ruthenium auf kristallinem Aluminosilikatton oder von Ruthenium-Zeolith-Katalysatoren sind aus der DE-OS 2 555 856 und der darin zitierten Patentliteratur bekannt.

Es ist bereits vorgeschlagen worden, die katalytische Hydrierung von Kohlehydraten in Gegenwart von suspendierten feinverteilten Ruthenium-Trägerkatalysatoren kontinuierlich durchzuführen (siehe US-PS 28 68 847 und DE-OS 2 555 856). Diese Arbeitsweise ist jedoch mit einer Reihe von schwerwiegenden Nachteilen behaftet.

Die Tatsache, daß der Katalysator bei Suspensionshydrierungen in feinverteilter Form eingesetzt wird, bedingt, daß der Kontakt durch aufwendige Filtrationseinrichtungen aus dem anfallenden Reaktionsgemisch entfernt werden muß. Die quantitative Abtrennung des Katalysators ist nicht nur zur Reinigung der Hydrierprodukte erforderlich. Beim Einsatz von Edelmetallkatalysatoren, wie im vorliegenden Fall, ist ihre praktisch quantitative Wiedergewinnung auch eine Grundvoraussetzung für die Wirtschaftlichkeit des Verfahrens. Im übrigen hat es sich gezeigt, daß die nach der Hydrierung abgetrennten Ruthenium-Trägerkatalysatoren bei der Wiederverwendung eine verminderte Aktivität zeigen, so daß sie spätestens nach dem dritten Durchgang regeneriert werden müssen.

Soll der abgetrennte Katalystor regeneriert oder zur Wiedergewinnung des Rutheniums aufgearbeitet werden, so muß er durch intensive Waschprozesse vom anhaftenden Hydrierungsprodukt befreit werden. Auch hier ist es ungünstig, daß der Katalysator als feines Pulver vorliegt. Weiterhin besteht bei der kontinuierlichen Hydrierung mit suspendierten Katalysatoren die Gefahr, daß Sedimentationsvorgänge zu Verstopfungen in den Reaktoren und den übrigen Teilen der Hydrieranlage führen. Schließlich verursacht die Abrasivität des Katalysator-Trägermaterials einen erhöhten Verschleiß der Anlagenteile, die mit der Suspension in Berührung kommen.

Demzufolge lag der Erfindung die Aufgabe zugrunde, ein kontinuierliches Verfahren zur Herstellung von Zuckeralkoholen aufzufinden, das frei von den geschilderten Nachteilen ist.

Die Lösung der Aufgabe ist ein Verfahren zur kontinuierlichen Herstellung von mehrwertigen Alkoholen durch Hydrierung von Kohlehydraten in Gegenwart von Ruthenium enthaltenden Katalysatoren bei erhöhter Temperatur und erhöhtem Druck, das dadurch gekennzeichnet ist, daß man ein Katalysatorfestbett aus stückigen Ruthenium-Trägerkatalysatoren einsetzt.

In Zusammenhang mit der Erfindung wird unter "Katalysatorfestbett" eine stationäre Anordnung des Katalysators im Reaktor in der Art einer Füllkörperschüttung verstanden. Die Durchführung von Hydrierungen an Festbettkatalysatoren ist an sich bekannt (siehe z.B. Ullmanns Enzyklopädie der technischen Chemie, 4. Auflage, Band 13, S. 135 ff.).

Für die Durchführung des erfindungsgemäßen Verfahrens sind alle Ruthenium enthaltenden festen Katalysatoren brauchbar, wie sie für die Hydrierung von Kohlehydraten zu den entsprechenden mehrwertigen Alkoholen eingesetzt werden. Hierbei handelt es sich inbesondere um Kontakte, in denen das katalytisch wirksame metallische Ruthenium in feinverteilter Form auf geeigneten Trägermaterialien wie Aluminiumoxid, Titandioxid, Kieselgur, Kieselgel, Molkularsiebe und Zeolithe natürlichen oder synthetischen Ursprungs niedergeschlagen ist. Bevorzugt werden Katalysatoren, deren Trägermaterial aus Aktivkohle besteht. Im Sinne der Erfindung werden diese Trägerkatalysatoren nicht wie bisher als feinverteiltes Pulver, sondern in kompaktierter stückiger Form eingesetzt.

Die in Betracht kommenden Trägerkatalysatoren enthalten in der Regel, bezogen auf das Gesamtgewicht des Kontaktes 0,1 bis 10 Gewichtsprozent, vorzugsweise 1 bis 4 Gewichtsprozent, elementares Ruthenium. Gewünschtenfalls können auch Katalysatoren mit einem höherem Gehalt an aktivem Metall eingesetzt werden.

Die Herstellung der Ruthenium enthaltenden Trägerkatalysatoren kann nach literaturbekannten Verfahren erfolgen. Beispielsweise kann man das Trägermaterial mit einer Rutheniumsalzlösung imprägnieren und dann das imprägnierte Material in Strom eines reduzierend wirkenden Gases trocknen und auf die Zersetzungstemperatur erhitzen.

Zur Verwendung im erfindungsgemäßen Verfahren werden die Ruthenium enthaltenden Trägerkatalysatoren durch an sich bekannte Verformungstechniken wie Tablettieren, Pelletieren oder Extrudieren, gegebenenfalls unter Zusatz von Bindemitteln, in stückige Form gebracht, beispielsweise in Kugel-, Zylinder- oder Hohlzylinderform.

Die Teilchengröße des in stückiger Form eingesetzten Katalysators kann in weiten Grenzen schwanken. Die Katalysatorpartikel sollten einerseits nicht so klein sein, daß der Strömungswiderstand des Katalysatorfestbetts den Durchfluß des Einsatzgemisches aus Wasserstoff und wäßriger Kohlehydratlösung stark behindert und einen zu hohen Druckaufwand erforderlich macht. Andererseits sind die maximalen Abmessungen des stückigen Ruthenium-Trägerkatalysators durch die Reaktorgeometrie vorgegeben. Bekanntlich sollte das Verhältnis von Reaktordurchmesser zum Durchmesser des Katalysatorkorns aus strömungstechnischen Gründen den Wert von etwa 7 bis 10 nicht unterschreiten (siehe J. Falbe und U. Hasserodt "Katalysatoren, Tenside und Mineralöladditive", Stuttgart 1978, Seite 21). In der Regel wird man den Durchmesser der Katalysatorpartikel so wählen, daß er im Bereich von 2 bis 10 mm liegt.

Als Ausgangsmaterial für das erfindungsgemäße Verfahren kommen Monosaccharide und deren Gemisch oder Polysaccharide enthaltende Materialien in Betracht. Der Ausdruck "Polysaccharide enthaltende Materialien" umfaßt dabei auch Disaccharide und deren Gemische sowie Kohlehydrate, die sowohl Monsaccharide als auch Polysaccharide enthalten. Beispiele für geeignete Monsaccharide sind Fructose, Galactose, Mannose, Arabinose, Ribose, Xylose und insbesondere Glucose. Die Pentosen und Hexosen sind die wichtigsten Monosaccharide. Das erfindungsgemäße Verfahren ist generell auf Monosaccharide mit mindestens 4 Kohlenstoffatomen, insbesondere auf solche mit 5 bis 7 Kohlenstoffatomen, anwendbar, wobei sowohl Zucker mit endständigen Aldehydgruppen (Aldosen) als auch solche mit einer innenständigen Ketogruppe (Ketosen) in Betracht kommen.

Als Beispiele für geeignete Disaccharide sind Lactose, Maltose, Saccharose, Cellobiose und Melibiose zu nennen. Ein geeignetes Trisaccharid ist Raffinose.

Weitere Polysaccharid enthaltende Ausgangsmaterialien sind Stärkeabbauprodukte, beispielsweise Dextrin, Glucosesirup und Stärkehydrolysate wie zum Beispiel Maisstärkehydrolysate, ferner Cellulosehydrolysate.

Die genannten Ausgangsmaterialien werden in Form von wässrigen Lösungen geeigneter Konzentration dem erfindungsgemäßen Verfahren unterworfen. Im allgemeinen wird die Hydrierung mit Lösungen im Konzentrationsbereich von 20 bis 80 Gewichtsprozent durchgeführt. Lösungen mit Konzentrationen im Bereich von 40 bis 70 Gewichtsprozent werden bevorzugt. Im übrigen ist es nicht unbedingt erforderlich, daß die zu hydrierenden Kohlehydrate mit dem Wasser echte Lösungen bilden; auch kolloidale Lösungen und Suspensionen von Kohlehydraten können dem erfindungsgemäßen Verfahren unterworfen werden.

Bei der Durchführung des erfindungsgemäßen Verfahrens wird die wäßrige Lösung des zu hydrierenden Kohlehydrats zusammen mit Wasserstoff kontinuierlich durch eine Reaktionszone geleitet, in der sich das Festbett aus stückigem Ruthenium-Trägerkatalysator befindet. Die Temperatur der Reaktionszone kann innerhalb weiter Grenzen variieren. Im allgemeinen kommen Reaktionstemperaturen zwischen 60 und 200 $^{\circ}$C in Betracht. Der Temperaturbereich von 90 bis 160 $^{\circ}$C wird bevorzugt. Vor allem bei der Hydrierung von Glucose empfiehlt es sich, Temperaturen von 160 $^{\circ}$C möglichst nicht zu überschreiten, da Glucose oberhalb dieser Temperatur zur Karamelisierung neigt. Im übrigen hat es sich als zweckmäßig erwiesen, die Mischung aus wäßriger Substratlösung und Wasserstoff bereits vor dem Eintritt in die Reaktionszone auf die vorgesehene Reaktionstemperatur zu erwärmen. Der Reaktionsdruck beträgt mindestens 25 bar und
kann Werte bis 500 bar annehmen. Reaktionsdrucke im
Bereich von 100 bis 300 bar werden bevorzugt.

Der pH-Wert der wäßrigen Substratlösungen liegt im
allgemeinen in der Nähe des Neutralpunktes. Dies gilt
vor allem für die Hydrierung von Monosacchariden. Wenn
bei der Hydrierung von Polysaccharid enthaltendem Ausgangsmaterial zusätzlich eine hydrolytische Spaltung der
Glukosidbindungen bewirkt werden soll, dann sollte der
pH-Wert der Substratlösung im Bereich von 2,5 bis 4,5,
vorzugsweise im Bereich von 3,0 bis 4,0 liegen. Die
Einstellung des pH-Wertes kann mit Mineralsäuren erfolgen, die in der erforderlichen Konzentration das
Reaktormaterial nicht angreifen, beispielsweise mit
Schwefelsäure oder Phosphorsäure. Selbstverständlich
kommen für die Durchführung des erfindungsgemäßen Verfahrens im sauren Bereich nur solche Rutheniumkatalysatoren in Betracht, deren Trägermaterial säurebeständig
ist. Es ist offensichtlich wichtig, daß die genannten
niederen pH-Werte in der letzten Reaktionsphase eingehalten werden. Aus diesem Grund kann es zweckmäßig sein,
die erforderliche Säure in der Mitte oder in der zweiten
Hälfte der Reaktionszone zuzuführen.

In entsprechenden Vergleichsversuchen konnte festgestellt werden, daß der Katalysatorbedarf beim erfindungsgemäßen Verfahren wesentlich geringer ist, als bei
der kontinuierlichen Durchführung des bekannten Suspensionshydrierverfahrens. Dieses Ergebnis war insofern
nicht vorauszusehen, als die beim Suspensionsverfahren
verwendeten Katalysatoren wegen ihrer geringen Partikelgröße einen höheren Porenausnutzungsgrad und damit eine größere Reaktivität zeigen sollten als die wesentlich grobkörnigeren Festbettkatalysatoren mit Partikeldurchmessern von 2 bis 10 mm, nachdem bekannt ist, daß der Poren-Ausnutzungsgrad normalerweise mit abnehmendem Katalysatorkorndurchmesser zunimmt. Außerdem sollte man für das Suspensionsverfahren ein engeres Verweilzeitspektrum gegenüber dem Suspensionsverfahren erwarten. Umso überraschender war demzufolge die Feststellung, daß das erfindungsgemäße Festbettverfahren im Vergleich zum Suspensionsverfahren wesentlich günstigere Werte für die Katalysatoraktivität und die mittlere Raum-Zeit-Ausbeute erbrachte.

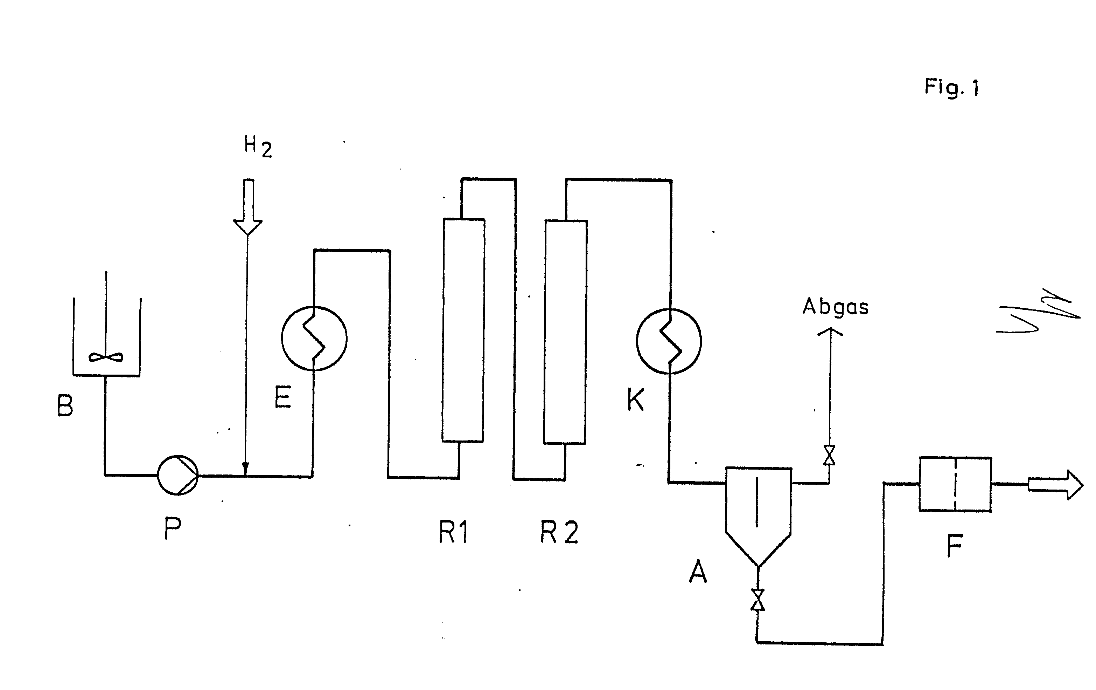

Die im folgenden beschriebenen Versuche wurden in einer Versuchsanlage gemäß Figur 1 durchgeführt. Die Elemente der Versuchsanlage sind: Vorratsbehälter B mit Rührer, Förderpumpe P, Erhitzer E, zwei hintereinander geschaltete Reaktoren R 1 und R 2, Kühler K, Abscheider A und Filtrationseinrichtung F. Die Reaktoren haben einen Inhalt von je 4,2 l; ihr Durchmesser ist 70 mm.

Der jeweilige Umsetzungsgrad wurde durch Bestimmen des Restgehaltes an reduzierenden Zuckern im Hydrierprodukt ermittelt. Hierzu wurden Proben des Reaktionsproduktes mit Wasser verdünnt, mit Fehlingscher Lösung erhitzt und mit Kaliumjodid versetzt. Anschließend wurde das durch Cu(I) freigesetzte Jod mit Natriumthiosulfatlösung titriert, wobei Stärkelösung als Indikator verwendet wurde.

## Vergleichsbeispiel

Die nachstehend beschriebene Hydrierung von Glucose wurde in der in Fig. 1 wiedergegebenen Apparatur durchgeführt. Als Katalysator wurde ein handelsüblicher Ruthenium-Trägerkatalysator (2 Gewichtsprozent Ru auf Aktivkohle) verwendet.

Eine 5o gewichtsprozentige Glucoselösung (d = 1,22), in der, auf Glucose bezogen, o,4 Gewichtsprozent Katalysator suspendiert waren, wurde aus dem Vorratsbehälter B mit Hilfe der Pumpe P in kontinuierlichem Strom zusammen mit Wasserstoff in den Erhitzer E gefördert, wo das Einsatzgemisch auf eine Temperatur von 15o$^\circ$ C gebracht wurde. Anschließend passierte das Gemisch aus Glucoselösung, Katalysator und Wasserstoff bei 15o$^\circ$ C und unter einem Druck von 25o bar, die aus zwei hintereinandergeschalteten Reaktoren R1 und R2 mit je 4,2 l Inhalt (Durchmesser 7o mm) bestehende Reaktionszone. Danach passierte das Reaktionsgemisch den Kühler K, in dem es auf 3o - 4o$^\circ$ C abgekühlt wurde, und gelangte in den Abscheider A, in dem der überschüssige Wasserstoff abgetrennt wurde. Nach dem Entspannen des Hydrierproduktes auf Atmosphärendruck wurde der Katalysator in der nachgeschalteten Filtrationseinrichtung F abgetrennt.

Bei einem Durchsatz von 4 l Glucoselösung pro Stunde und einer stündlichen $H_2$-Entspannung von 12 Nm$^3$ wurde ein 99 %iger Umsatz der Glucose erzielt.

Der abfiltrierte Katalysator wurde ein zweites und ein drittes Mal eingesetzt. Beim zweiten Durchgang wurde eine leicht verminderte Aktivität des Katalysators beobachtet; unter den gleichen Bedingungen wie beim ersten Durchgang wurde ein 95 %iger Umsatz der Glucose erreicht. Beim drit-

...

0082277

ten Durchgang zeigte der Katalysator einen drastischen Aktivitätsabfall; es wurde nur noch ein 25 %iger Glucoseumsatz erzielt.

Danach läßt sich der eingesetzte Rutheniumkatalysator unter den Bedingungen der Suspensionshydrierung ohne Regenerierung praktisch nur einmal wiederverwenden.

Definiert man die mittlere Aktivität des Katalysators $\bar{a}_k$ als die in beiden Durchgängen pro Mengeneinheit Katalysator gebildete Menge an Hydrierprodukt Sorbit und legt man für beide Durchgänge einen mittleren Umsatz von 97 % zugrunde, so ergibt sich für das Suspensionsverfahren ein Wert von

$$\bar{a}_k = \frac{2 \times 0,97 \times 100 \times MG(\text{Sorbit})}{0,4 \times MG\ (\text{Glucose})} = 490$$

Umgekehrt errechnet sich für den Katalysatorbedarf, bezogen auf die in beiden Durchgängen insgesamt aus Glucose gewonnene Menge Sorbit, ein Wert von 0,204 Gewichtsprozent. Berücksichtigt man den Edelmetallgehalt des Trägerkatalysators, so ergibt sich ein Rutheniumbedarf von $4,1 \cdot 10^{-3}$ Gewichtsprozent.

Beispiel 1

Für die im folgenden beschriebenen erfindungsgemäßen Hydrierungen wurde die Apparatur nach Fig. 1 so modifiziert, daß das Gemisch Zuckerlösung, Katalysator und Wasserstoff nach dem Passieren des Erhitzers E jeweils von oben durch die hintereinandergeschalteten Reaktoren R1 und R2 geleitet wurde. Außerdem wurde die Filtrationsanlage F entfernt.

...

Als Katalysator wurde ein handelsüblicher kompaktierter Ruthenium-Trägerkatalysator (2 Gewichtsprozent Ru auf Tierkohle, zylindrische Form, Durchmesser 2 mm, Länge 2-5 mm) verwendet. Die beiden Reaktoren R1 und R2 wurden mit insgesamt 3,12 kg stückigem Katalysator gefüllt.

Eine 5o gewichtsprozentige Glucoselösung (d = 1,22) wurde aus dem Vorratsbehälter B über die Pumpe P in kontinuierlichem Strom zusammen mit Wasserstoff in den Erhitzer E gefördert, wo das Gemisch auf 15o° C erhitzt wurde. Danach passierte das Gemisch bei 15o° C und 25o bar die mit stückigem Katalysator gefüllten Reaktoren R1 und R2. Anschließend wurde das Reaktionsgemisch im Kühler K auf 3o - 4o° C abgekühlt und schließlich im Abscheider A vom überschüssigen Wasserstoff getrennt.

Der Durchsatz der Glucoselösung wurde so gewählt, daß der Glucosegehalt des Hydrierproduktes o,5 Gewichtsprozent nicht überstieg. Dies entspricht einem Umsatz von 99 %. Zu Beginn der Betriebsperiode lag der Durchsatz bei 8 l/h. Im Verlauf des Versuchs wurde der Durchsatz bis auf 3 l/h vermindert. Nach einer Betriebszeit von 155o Stunden wurde der Versuch beendet. Es wurden insgesamt 52 oo kg Glucose umgesetzt; die Sorbitausbeute betrug 5205 kg.

Die pro Mengeneinheit Katalysator gebildete Sorbitmenge (mittlere Aktivität des Katalysators $\bar{a}_k$) betrug demnach 1668 und war demnach um den Faktor 3,4 höher als bei der Suspensionshydrierung im Vergleichsversuch. Dementsprechend liegt der Katalysatorbedarf für die Herstellung von Sorbit nach dem erfindungsgemäßen Verfahren bei o,06 Gewichtsprozent oder, bezogen auf Ruthenium, bei $1,2 \cdot 1o^{-3}$ Gewichtsprozent.

...

0082277

Der über die Gesamtfahrzeit ermittelte Durchsatz an 5o gewichtsprozentiger Glucoselösung lag im Falle der erfindungsgemäßen Festbetthydrierung bei 5,5 l/h, d. h. um 37,5 % höher als bei der Suspensionshydrierung nach dem Stand der Technik.

Beispiel 2

Die in Beispiel 1 beschriebene Verfahrensweise wurde so abgewandelt, daß der Durchsatz an 5o gewichtsprozentiger Glucoselösung konstant bei 4 l/h belassen wurde. Die Reaktionstemperatur wurde im Verlauf des Versuches von anfänglich 1oo° C in Stufen von jeweils 1o° C in der Weise angehoben, daß mindestens ein Umsatz von 99 % erreicht wurde. Die Endtemperatur war 16o° C. Während des ganzen Versuches wurde ein Wasserstoffdruck von 1oo bar aufrecht erhalten. Bei einem Einsatz von 2,96 kg Katalysator wurden insgesamt 882o l Glucoselösung (538o kg Glucose) durchgesetzt. Die Sorbitausbeute betrug 5386 kg. Die für die Herstellung von Sorbit aus Glucose benötigte Rutheniummenge betrug demnach $1,1 \cdot 1o^{-3}$ Gewichtsprozent, bezogen auf erhaltenen Sorbit.

Beispiel 3

In Analogie zu Beispiel 1 wurde Invertzucker in 5o gewichtsprozentiger Lösung bei 1oo° C unter einem Wasserstoffdruck von 25o bar katalytisch hydriert. Dabei wurden bezüglich Durchsatz und Polyolausbeute mit der Glucosehydrierung vergleichbare Ergebnisse erzielt.

Beispiel 4

In Analogie zu Beispiel 1 wurde ein Maisstärkehydrolysat (DE = 92) in 4o gewichtsprozentiger Lösung bei 15o° C

...

unter einem Wasserstoffdruck von 25o bar katalytisch hydriert. Bei einem durchschnittlichen Durchsatz von 5 l/h wurde ein Umsetzungsgrad von 98 %, bezogen auf den Gehalt an reduzierenden Zuckern erreicht. Der Rutheniumbedarf bei der Hydrierung des Maisstärkehydrolysats lag bei $1,3 \cdot 10^{-3}$ Gewichtsprozent, bezogen auf erhaltene Polyole.

## Patentanspruch

Verfahren zur kontinuierlichen Herstellung von mehrwertigen Alkoholen durch Hydrierung von Kohlehydraten
in Gegenwart von Ruthenium enthaltenden Katalysatoren
bei erhöhter Temperatur und erhöhtem Druck, dadurch
gekennzeichnet, daß man ein Katalysatorfestbett aus
stückigen Ruthenium-Trägerkatalysatoren einsetzt.

Fig. 1

0082277

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| | --- | | C 07 C 31/18 |
| A | DE-A-2 536 416 (ICI UNITED STATES) <br> * Ansprüche 1, 4 * | 1 | C 07 C 29/136 <br> C 07 C 29/14 |
| | --- | | |
| A,D | DE-A-2 555 856 (ICI UNITED STATES) <br> * Anspruch 1 * | 1 | |
| | --- | | |
| A | US-A-4 072 628 (W.M. KRUSE et al.) <br> * Ansprüche 1, 7 * | 1 | |
| | --- | | |
| A | FR-A-1 600 072 (HENKEL) <br> * Anspruch 1 * | 1 | |
| | --- | | **RECHERCHIERTE SACHGEBIETE (Int. Cl. ³)** |
| A | INSPEC PATENT ASSOCIATED LITERATURE, B600-7502-I & Bullentin of the Academy of Sciences USSR: Division of Chemical Science N.A. VASYUNINA et al. "Hydrolytic hydrogenation of saccharose" Seiten 1506-1507 (Izvestiya Akademii Nauk SSSR Seriya Khimicheskaya Band 23,1975, Nr. 7 | 1 | B 01 J 23/46 <br> B 01 J 35/02 <br> C 07 C 29/136 <br> C 07 C 29/14 <br> C 07 C 31/18 <br> C 07 C 31/24 <br> C 07 C 31/26 |
| | ----- | | |

Der vorliegende Recherchenbericht wurde für alle Patentansprüche erstellt.

| Recherchenort <br> BERLIN | Abschlußdatum der Recherche <br> 03-02-1983 | Prüfer <br> KNAACK M |
|---|---|---|